# EUROPEAN PATENT APPLICATION

(11) **EP 3 813 061 A1**
(43) Date of publication of application: **28.04.2021**
(21) Application number: 18923160.8
(22) Date of filing: 21.06.2018
(51) Int. Cl.: G10L 15/10, G10L 25/51, G10L 25/66

(54) **ATTRIBUTE IDENTIFYING DEVICE, ATTRIBUTE IDENTIFYING METHOD, AND PROGRAM STORAGE MEDIUM**

(71) Applicant: NEC Corporation, 108-8001 Tokyo (JP)
(72) Inventor: YAMAMOTO Hitoshi, Tokyo 108-8001 (JP); KOSHINAKA Takafumi, Tokyo 108-8001 (JP)
(74) Representative: Betten & Resch
(86) International application number: PCT/JP2018/023594
(87) International publication number: WO 2019/244298

(57) **Abstract**

To provide an attribute identifying device, an attribute identifying method, and a program storage medium in which the accuracy of attribute identification of a person is further enhanced.

An attribute identifying device 100 includes a first attribute identifying unit 130 that identifies, based on a biological signal, first attribute information, which is a range of specific attribute values, from the biological signal, and a second attribute identifying unit 140 that identifies second attribute information, which is specific attribute information, from the biological signal and the first attribute information.

## Description

### [Technical Field]

The example embodiments relates to an attribute identifying device, an attribute identifying method, and a program storage medium.

### [Background Art]

A voice processing device that estimates attribute information such as the sex or age of a person from a biological signal such as a voice signal obtained from an utterance of a speaker is known.

When estimating the attribute information of a person, the voice processing device of this type may estimate the attribute information as a discrete value or the attribute information as a continuous value.

PTL 1 describes a technique for estimating the age as an attribute of a person from a face image signal. In the age estimation technique described in PTL 1, first, the age is estimated as a discrete value from the face image signal, and the age is estimated as a continuous value. In the age estimation technique described in PTL 1, the estimation results by the above discrete value and continuous value are integrated to calculate a final estimated value.

### [Citation List]

### [Patent Literature]

[PTL 1] JP 4273359 B2

### [Non Patent Literature]

[NPL 1] Najim Dehak, Patrick Kenny, Reda Dehak, Pierre Dumouchel, Pierre Ouellet, "Front-End Factor Analysis for Speaker Verification" IEEE Transaction on Audio, Speech and Language Processing, Volume 19, No. 4, pp. 788-798, 2011.

### [Summary]

### [Technical Problem]

However, the technique described in PTL 1 has a problem that the accuracy of identifying the attribute of a person is not sufficient.

In the technique described in PTL 1, when the age is estimated as the attribute of a person from the face image signal, a first estimated value, which is a discrete value, and a second estimated value, which is a continuous value, are integrated in accordance with a previously designed rule to calculate the final estimated value. The technique described in PTL 1 independently obtains the first estimated value and the second estimated value. Therefore, there is a case where the first estimate value and the second estimated value are greatly different, and in this case, two estimated values seem correct even after the integration, and it is difficult to narrow down the two estimated values to a single estimated value. Therefore, the identification accuracy of the age may be impaired.

The example embodiments has been made in view of the above problem, and an object thereof is to provide an attribute identifying device, an attribute identifying method, and a program storage medium in which the accuracy of attribute identification of a person is further enhanced.

### [Solution to Problem]

An attribute identifying device according to one aspect of the example embodiments includes a first attribute identifying means identifying, based on a biological signal, first attribute information, which is a range of specific attribute values, from the biological signal, and a second attribute identifying means identifying second attribute information, which is specific attribute information, from the biological signal and the first attribute information.

An attribute identifying method according to one aspect of the example embodiments includes identifying, based on a biological signal, first attribute information, which is a range of specific attribute values, from the biological signal, and identifying second attribute information, which is specific attribute information, from the biological signal and the first attribute information.

A program storage medium according to one aspect of the example embodiments stores a program that causes a computer to execute a process of identifying, based on a biological signal, first attribute information, which is a range of specific attribute values, from the biological signal, and a process of identifying second attribute information, which is specific attribute information, from the biological signal and the first attribute information.

### [Advantageous Effects]

According to the example embodiments, it is possible to provide an attribute identifying device, an attribute identifying method, and a program storage medium in which the accuracy of attribute identification of a person is further enhanced.

### [Brief Description of Drawings]

Fig. 1 is a block diagram illustrating a hardware configuration of a computer device for implementing a device according to each example embodiment.
Fig. 2 is a block diagram illustrating a functional configuration of a voice processing device according to a first example embodiment.
Fig. 3 is a diagram for describing an example of first attribute information output by a first attribute identifying unit of the voice processing device according to the first example embodiment.
Fig. 4 is a diagram for describing another example of a second attribute identifying unit of the voice processing device according to the first example embodiment.
Fig. 5 is a diagram for describing another example of the first attribute information output by the first attribute identifying unit of the voice processing device according to the first example embodiment.
Fig. 6 is a flowchart illustrating an operation of the voice processing device according to the first example embodiment.
Fig. 7 is a block diagram illustrating a functional configuration of an attribute identifying device according to an example embodiment having a minimum configuration.

### [Example Embodiment]

Example embodiments will be described below with reference to the drawings. It should be noted that in the example embodiments, components denoted by the same reference signs perform similar operations, and thus, the description thereof may be omitted. Directions of arrows in the drawings are illustrative and do not limit directions of signals between blocks.

### <First Example Embodiment>

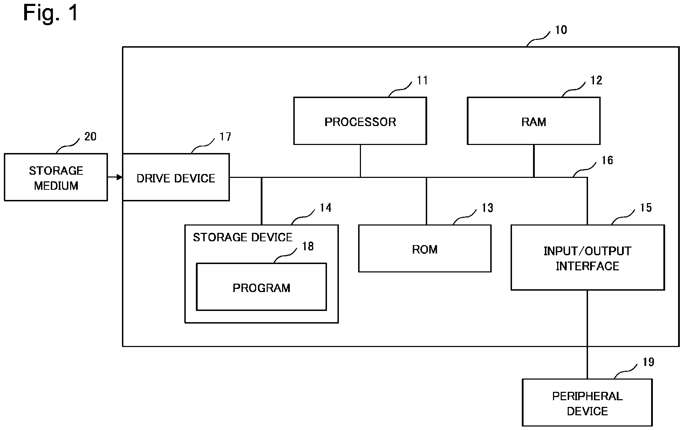

Hardware constituting a voice processing device or an attribute identifying device according to a first example embodiment and another example embodiment will be described. Fig. 1 is a block diagram illustrating a hardware configuration of a computer device 10 for implementing the voice processing device and a voice processing method according to each example embodiment. In each example embodiment, components of the voice processing device shown below indicate blocks of functional units. Each component of the voice processing device can be implemented by any combination of the computer device 10 as illustrated in Fig. 1 and software, for example.

As illustrated in Fig. 1, the computer device 10 includes a processor 11, a random access memory (RAM) 12, a read only memory (ROM) 13, a storage device 14, an input/output interface 15, and a bus 16.

The storage device 14 stores a program 18. The processor 11 uses the RAM 12 to execute the program 18 associated with the voice processing device. The program 18 may be stored in the ROM 13. The program 18 may be stored on a storage medium 20 and read out by a drive device 17 or transmitted from an external device via a network.

The input/output interface 15 exchanges data with a peripheral device (keyboard, mouse, display device, or the like) 19. The input/output interface 15 may function as a means acquiring or outputting data. The bus 16 connects the components.

It should be noted that there are various modified examples of a method for implementing the voice processing device. For example, each unit of the voice processing device can be implemented as hardware (dedicated circuit). In addition, the voice processing device can be implemented by a combination of a plurality of devices.

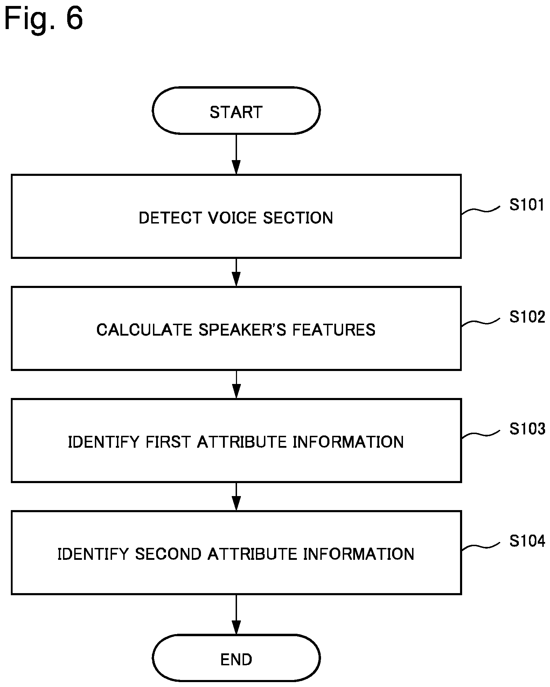

The scope of each example embodiment also includes a processing method in which a program for operating the configuration of each example embodiment so as to implement functions of the present example embodiment and the another example embodiment (more specifically, a program that causes a computer to execute processing illustrated in Fig. 6 or the like) is stored on a storage medium, the program stored in the storage medium is read out as a code, and the program is executed in the computer. That is, a computer-readable storage medium is also included in the scope of each example embodiment. Not only the storage medium on which the above-described program is stored, but also the program itself is included in each example embodiment.

As the storage medium, for example, a floppy (registered trademark) disk, a hard disk, an optical disk, a magneto-optical disk, a compact disc (CD)-ROM, a magnetic tape, a nonvolatile memory card, or a ROM can be used. In addition, the scope of each example embodiment includes not only a method that executes the processing by using only the program stored on the storage medium, but also a method that executes the processing by operating the program on an operating system (OS) in cooperation with other software or expansion board functions.

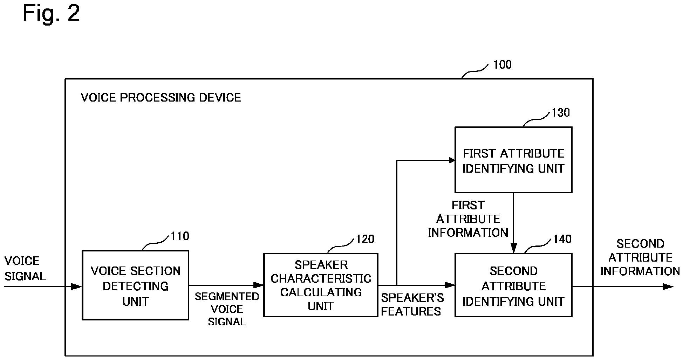

Fig. 2 is a block diagram illustrating a functional configuration of a voice processing device 100 according to the first example embodiment. As illustrated in Fig. 2, the voice processing device 100 includes a voice section detecting unit 110, a speaker feature vector calculating unit 120, a first attribute identifying unit 130, and a second attribute identifying unit 140.

The voice section detecting unit 110 receives a voice signal from the outside. The voice signal is a signal representing a voice based on an utterance of a speaker. The acquired signal is not limited to the voice signal, but may be a biological signal generated from a body by a biological phenomenon such as heartbeat, brain wave, pulse, respiration, or sweating.

The voice section detecting unit 110 detects and segments a voice section included in the received voice signal. At this time, the voice section detecting unit 110 may segment the voice signal into voice signals having predetermined lengths or into voice signals having different lengths. For example, the voice section detecting unit 110 may determine that a section of the voice signal in which the volume is lower than a predetermined value continuously for a predetermined period of time is silent, and may determine that sections before and after the section are different voice sections, so as to perform segmentation. The voice section detecting unit 110 outputs the segmented voice signals as segmentation results (processing results of the voice section detecting unit 110) to the speaker feature vector calculating unit 120. Here, the reception of the voice signal means example, reception of the voice signal from an external device or another processing device, or delivery of processing results of voice signal processing from another program. The output means example, transmission to an external device or another processing device, or delivery of the processing results of the voice section detecting unit 110 to another program.

The speaker feature vector calculating unit 120 receives the segmented voice signals from the voice section detecting unit 110. The speaker feature vector calculating unit 120 calculates speaker feature vectors expressing feature vectors of individuality included in the segmented voice signals on the basis of the received segmented voice signals. The speaker feature vector calculating unit 120 outputs the calculated speaker feature vectors (processing results of the speaker feature vector calculating unit 120).

That is, the speaker feature vector calculating unit 120 serves as a speaker feature vector calculating means calculating speaker feature vectors representing the individuality of a speaker on the basis of a voice signal representing a voice, which is a biological signal. Hereinafter, speaker feature vectors calculated for a certain voice signal are referred to as speaker feature vectors of the voice signal.

An example of the speaker feature vectors calculated by the speaker feature vector calculating unit 120 will be described. The speaker feature vector calculating unit 120 calculates a feature vector vector based on an i-vector representing the individuality of a voice quality of a speaker on the basis of the segmented voice signals received from the voice section detecting unit 110. The speaker feature vector calculating unit 120 may use, for example, a method described in NPL 1 as a method for calculating the feature vector vector based on the i-vector representing the individuality of the voice quality of the speaker. It should be noted that the speaker feature vectors calculated by the speaker feature vector calculating unit 120 are vectors that can be calculated by a predetermined operation on the segmented voice signals, and may be feature vectors representing the individuality of the speaker, and the i-vector is an example thereof.

Another example of the speaker feature vectors calculated by the speaker feature vector calculating unit 120 will be described. The speaker feature vector calculating unit 120 calculates a feature vector vector representing frequency analysis results of the voice signal on the basis of the segmented voice signals received from the voice section detecting unit 110. The speaker feature vector calculating unit 120 calculates, as feature vectors representing the frequency analysis results, frequency filter bank feature vectors obtained by fast Fourier transform (FFT) processing and filter bank processing, or mel frequency cepstral coefficient (MFCC) feature vectors obtained by discrete cosine transform processing in addition to the above processing, for example.

The first attribute identifying unit 130 receives the speaker feature vectors output by the speaker feature vector calculating unit 120. The first attribute identifying unit 130 estimates (identifies) specific attribute information by using the speaker feature vectors and outputs the attribute information as first attribute information. The specific attribute information may be, for example, information indicating an age group of a speaker. The first attribute identifying unit 130 serves as a first attribute identifying means identifying, on the basis of the biological signal, first attribute information, which is a range of specific attribute values, from the biological signal. The identification includes estimation of an attribute value, classification based on a range of the attribute values, and the like.

An example of a method in which the first attribute identifying unit 130 estimates the first attribute information will be described. The first attribute identifying unit 130 may use, for example, a neural network as an identifier. The first attribute identifying unit 130 may use, as the identifier, a probabilistic model such as a Gaussian mixture distribution, or an identification model such as a linear discriminant analysis or a support vector machine. Here, the identifier of the first attribute identifying unit 130 learns learning data in which speaker feature vectors related to voice signals are associated with classes (details will be described later) including attribute values of a speaker. The learning generates the identifier whose input is the speaker feature vectors and whose output is a class (first attribute information). For example, when the neural network is used as the identifier, the first attribute identifying unit 130 calculates attribute information to be output on the basis of the speaker feature vectors of the input and a weighting coefficient of the neural network.

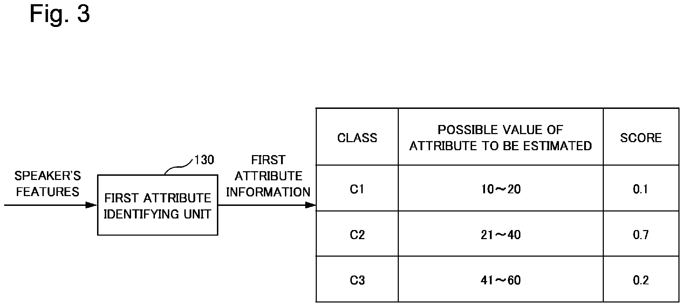

Fig. 3 is a diagram for describing an example of the first attribute information output by the first attribute identifying unit 130. The example of the first attribute information output by the first attribute identifying unit 130 will be described with reference to Fig. 3. The first attribute identifying unit 130 defines classes on the basis of a range of possible values of an attribute to be estimated, scores the classes, and outputs, as the first attribute information, a vector having the scores as values. Here, the score is a value indicating correlation between a result calculated by the identifier and attribute information to be estimated. That is, the score is a value indicating the certainty of the estimation result calculated by the identifier.

The first attribute identifying unit 130 defines the classes on the basis of the range of possible values of the attribute to be estimated. Here, it is assumed, for example, that the possible value of the attribute to be estimated is a natural number from "10" to "60". At this time, as illustrated in Fig. 3, the first attribute identifying unit 130 defines, for example, a class including "10" to "20" as C1, a class including "21" to "40" as C2, and a class including "41" to "60" as C3. The first attribute identifying unit 130 then scores the classes calculated by the identifier, and outputs, as an estimated value of the classes C1 to C3, the vector having the scores as values. As illustrated in Fig. 3, the first attribute identifying unit 130 calculates, for example, scores of the classes C1, C2, and C3 as 0.1, 0.7, and 0.2, respectively. At this time, for example, the first attribute identifying unit 130 outputs, as the estimated value, a vector V1 = (0.1, 0.7, 0.2) having scores associated to the above classes C1 to C3 as components. The first attribute identifying unit 130 may also output, as the estimated value, a vector whose value is the number of one class. For example, the first attribute identifying unit 130 may output a vector whose value is the number of a class with the highest score among the scores that are vector components, as a vector V2 = (2).

The second attribute identifying unit 140 receives the speaker feature vectors output by the speaker feature vector calculating unit 120 and the first attribute information output by the first attribute identifying unit 130. The second attribute identifying unit 140 estimates (identifies) specific attribute information (second attribute information) by using the received speaker feature vectors and first attribute information. The specific attribute information may be, for example, information indicating the age of a speaker. The second attribute identifying unit 140 serves as a second attribute identifying means identifying second attribute information, which is the specific attribute information, from the biological signal and the first attribute information.

An example of a method in which the second attribute identifying unit 140 estimates the specific attribute information will be described. The second attribute identifying unit 140 may use, for example, a neural network as an identifier.

Here, the identifier of the second attribute identifying unit 140 learns learning data in which speaker feature vectors related to voice signals, attribute values of a speaker, and classes including the attribute values are associated. The learning generates the identifier whose input is the speaker feature vectors and the first attribute information, which is the output of the first attribute identifying unit 130 to which the speaker feature vectors are input, and whose output is the attribute information (attribute value), which is an estimation result. When the neural network is used as the identifier, the second attribute identifying unit 140 calculates the attribute information to be output on the basis of the input including the speaker feature vectors and the first attribute information and a weighting coefficient of the neural network.

At this time, the second attribute identifying unit 140 calculates the estimation result as a continuous value.

As described above, the second attribute identifying unit 140 can enhance the accuracy of attribute identification by using, as the input, the first attribute information output by the first attribute identifying unit 130. The reason for this is that the second attribute identifying unit 140 estimates the attribute information by using, as prior information, the result estimated by the first attribute identifying unit 130, so that there is a high possibility of outputting a value close to a true value, as compared with estimation only from the speaker feature vectors without the prior information. In particular, when the second attribute identifying unit 140 estimates a continuous value, since the identifier learns to minimize a residual at a learning stage, the estimated value tends to be biased toward the middle of the overall values if the overall performance is to be improved. That is, when the true value is lower than an average value, the estimated value is likely to be estimated higher, and when the true value is higher than the average value, the estimated value is likely to be estimated lower. On the other hand, when the range of the attribute values estimated by the first attribute identifying unit 130 is used as the prior information, the above-described bias can be reduced.

Here, the second attribute identifying unit 140 may calculate the estimation result as a discrete value. In this case, the second attribute identifying unit 140 calculates, as the estimation result by the discrete value, a class whose value range is narrower (more limited) than that of the class defined by the first attribute identifying unit 130. The identifier of the second attribute identifying unit 140 learns learning data in which speaker feature vectors related to input voice signals and classes including attribute values of a speaker are associated. At this time, the second attribute identifying unit 140 uses, for the learning data, classes each defined in a range narrower than the range of the attribute values defined by the first attribute identifying unit 130. In a case of the above-described example, the range of values included in each of the classes C1 to C3 and classes D1 to D3 defined by the first attribute identifying unit 130 is "10". Therefore, the second attribute identifying unit 140 defines the classes in units of "5", for example, so that the range is narrower than "10". The second attribute identifying unit 140 uses the classes defined in this way for the learning data. The learning generates the identifier whose input is the speaker feature vectors and the first attribute information, which is the output of the first attribute identifying unit 130 to which the speaker feature vectors are input, and whose output is the attribute information (class), which is the estimation result.

When the above model is used, the second attribute identifying unit 140 calculates the estimation result as a discrete value.

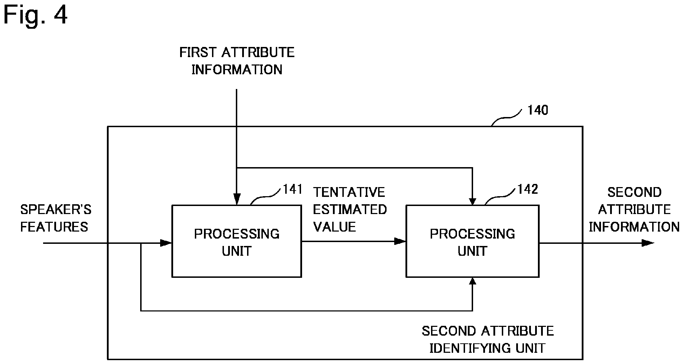

The second attribute identifying unit 140 may have a multi-stage configuration. Fig. 4 is a diagram for describing an example of the second attribute identifying unit 140 having the multi-stage configuration. As illustrated in Fig. 4, the second attribute identifying unit 140 may include a processing unit 141 that performs discrimination analysis and a processing unit 142 that performs regression analysis.

In this case, the second attribute identifying unit 140 calculates a discrete value as a tentative estimated value (tentative attribute information) in the processing unit 141, and uses the tentative estimated value to calculate an estimated value as a continuous value by the processing unit 142.

The processing unit 141 learns learning data in which speaker feature vectors related to voice signals are associated with classes including attribute values of a speaker. The learning generates an identifier whose input is the speaker feature vectors and the output of the first attribute identifying unit 130 to which the speaker feature vectors are input, and whose output is a class (tentative estimated value). At this time, the processing unit 141 calculates the tentative estimated value indicated by a class in a unit of "5", for example, as described above, by using the speaker feature vectors and the first attribute information.

The processing unit 142 learns learning data in which speaker feature vectors related to voice signals, attribute values of a speaker, and classes including the attribute values of the speaker are associated. The learning generates an identifier whose input is the speaker feature vectors, the output (tentative estimated value) of the processing unit 141 to which the speaker feature vectors are input, and the first attribute information, and whose output is the second attribute information (attribute value), which is the estimation result.

The processing unit 142 calculates the estimated value by a continuous value by using the speaker feature vectors, the first attribute information, and the tentative estimated value, which is the output of the processing unit 141. The processing unit 142 may also use the tentative estimated value calculated by the processing unit 141 to calculate and output, as a discrete value, a class defined in a range narrower than a range of the attribute values defined by the processing unit 141.

As described above, the second attribute identifying unit 140 defines the range of the attribute values that is narrower (finer) than the range of the attribute values defined by the first attribute identifying unit 130, and estimates the class. Alternatively, the second attribute identifying unit 140 estimates the attribute value as a continuous value. Therefore, it can be said that the second attribute identifying unit 140 has a function capable of outputting the true value. Although the voice processing device 100 includes a plurality of attribute identifying units, a single estimated value can be calculated because the second attribute identifying unit 140 calculates a final estimated value. As described above, in the voice processing device 100, the second attribute identifying unit 140 calculates the attribute information by using the first attribute information output by the first attribute identifying unit 130 in addition to the speaker feature vectors, so that the attribute estimation result with high accuracy can be output.

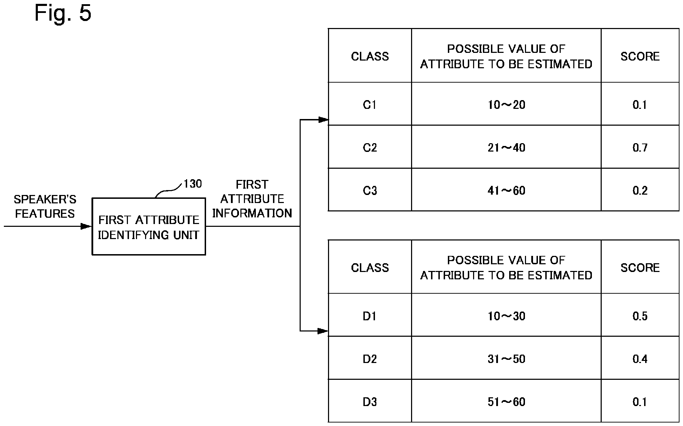

It should be noted that in the above description, the first attribute identifying unit 130 outputs one piece of attribute information, but the first attribute identifying unit 130 may output a plurality of pieces of attribute information. Fig. 5 is a diagram for describing another example of the first attribute information output by the first attribute identifying unit 130. As illustrated in Fig. 5, the first attribute identifying unit 130 may define groups of classes on the basis of a range of possible values of the attribute to be estimated and calculate and output an estimated value for each group. The groups of classes include ranges of values different from each other. As illustrated in Fig. 5, the first attribute identifying unit 130 defines, as ranges different from those of the above classes C1 to C3, a class including "10" to "30" as D1, a class including "31" to "50" as D2, and a class including "51" to "60" as D3, for example. The first attribute identifying unit 130 scores the classes D1 to D3 similarly to the above manner, and outputs a vector having the scores as values, together with the vector V1. In the example illustrated in Fig. 5, the first attribute identifying unit 130 outputs the vector V1 = (0.1, 0.7, 0.2) and a vector V3 = (0.5, 0.4, 0.1). If the identifier used by the first attribute identifying unit 130 is the neural network, the first attribute identifying unit 130 may be configured in which one identifier includes two output layers associated to the classes C1 to C3 and the classes D1 to D3, respectively.

As described above, the first attribute identifying unit 130 can enhance the accuracy of attribute identification by defining a plurality of classes such that the classes adopt different ways of dividing the range of possible values of the attribute to be estimated. For example, in the case of identifying which of the classes C1 to C3 includes the attribute value, the class C2 including "21" to "40" is focused on. In this case, the identification accuracy is lower at "21" and "40" close to boundaries of the range of values included in C2 than at "30" close to the middle of the range of values included in C2. That is, "21" may be identified as an incorrect one of the class C1 and C2, and "40" may be identified as an incorrect one of the class C2 and C3. Therefore, as described above, the classes D1 to D3 are separately defined as ranges of values in which values close to the boundaries such as "21" and "40" are close to the middle. That is, the first attribute identifying unit 130 divides the attribute values in two or more patterns so that boundary values in ranges of the attribute values are different from each other, and identifies a range of the attribute values in each of the divisions. As a result, values close to boundaries in the classes C1 to C3 can be identified similarly to values close to the middle, so that the identification accuracy can be enhanced.

As described above, in the voice processing device 100 according to the present example embodiment, the first attribute identifying unit 130 roughly estimates the attribute value as the first attribute information, and the second attribute identifying unit 140 estimates the attribute value in detail by using the first attribute information. Thus, according to the present example embodiment, the attribute value can be accurately estimated for the voice signal. That is, the voice processing device 100 according to the present example embodiment can enhance the accuracy of attribute identification of a person.

### <Operation of First Example Embodiment>

Next, an operation of the voice processing device 100 according to the first example embodiment will be described with reference to a flowchart of Fig. 6. Fig. 6 is the flowchart illustrating an example of the operation of the voice processing device 100.

The voice processing device 100 receives one or more voice signals from the outside and provides the voice signals to the voice section detecting unit 110. The voice section detecting unit 110 segments the received voice signals and outputs the segmented voice signals to the speaker feature vector calculating unit 120 (step S101).

The speaker feature vector calculating unit 120 calculates speaker feature vectors for each of the received one or more segmented voice signals (step S102).

The first attribute identifying unit 130 identifies and outputs first attribute information on the basis of the received one or more speaker feature vectors (step S103).

The second attribute identifying unit 140 identifies and outputs second attribute information on the basis of the received one or more speaker feature vectors and first attribute information (step S104). When the reception of the voice signals from the outside is completed, the voice processing device 100 ends the series of processing.

### <Effect of First Example Embodiment>

As described above, according to the voice processing device 100 according to the present example embodiment, the accuracy of attribute identification of a person can be improved. This is because the voice processing device 100 uses the first attribute information roughly estimated by the first attribute identifying unit 130 to estimate and output the attribute information in more detail by the second attribute identifying unit 140.

As described above, according to the voice processing device 100 according to the present example embodiment, the estimated value can be obtained with a certain accuracy regardless of the possible value of the attribute, by a calculation method in which the attribute identification of a person is calculated in a gradually detailed manner.

The voice processing device 100 according to the first example embodiment is an example of an attribute identifying device that identifies specific attribute information from a voice signal. The voice processing device 100 can be used as an age identifying device when the specific attribute information is the age of a speaker. The attribute information may be information indicating the sex of a speaker, information indicating an age group of a speaker, or information indicating the physique of a speaker.

The voice processing device 100 can be used as an emotion identifying device when the specific attribute information is information indicating the emotion of a speaker during an utterance. The voice processing device 100 can also be used, for example, as a part of a voice retrieval device or a voice display device provided with a mechanism for specifying one of a plurality of stored voice signals associated to a specific feeling, on the basis of emotion information estimated by use of emotion feature vectors. The emotion information includes, for example, information indicating emotion expressions and information indicating the character of a speaker.

### <Second Example Embodiment>

An example embodiment having a minimum configuration of the embodiment will be described.

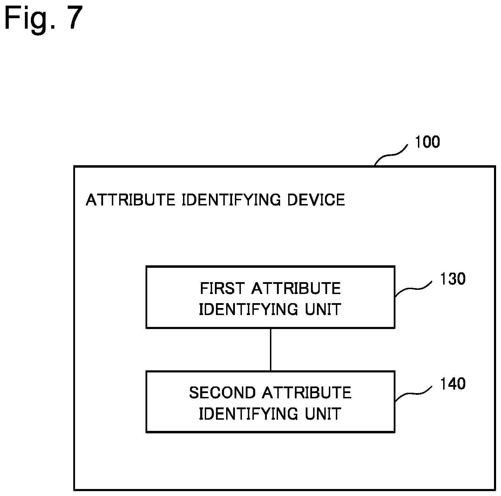

Fig. 7 is a block diagram illustrating a functional configuration of an attribute identifying device 100 according to the example embodiment having the minimum configuration. As illustrated in Fig. 7, the attribute identifying device 100 includes a first attribute identifying unit 130 and a second attribute identifying unit 140.

The first attribute identifying unit 130 identifies, on the basis of a biological signal, first attribute information, which is a range of specific attribute values, from the biological signal. The second attribute identifying unit 140 identifies second attribute information, which is specific attribute information, from the biological signal and the first attribute information.

According to the second example embodiment, by adopting the above configuration, the second attribute identifying unit 140 uses, as an input, the first attribute information output by the first attribute identifying unit 130, and thus it is possible to obtain an effect that the accuracy of attribute identification of a person can be further enhanced.

While the embodiments has been particularly shown and described with reference to example embodiments thereof, the embodiments is not limited to these example embodiments. It will be understood by those of ordinary skill in the art that various changes in form and details may be made therein without departing from the spirit and scope of the example embodiments as defined by the claims. That is, the embodiments is not limited to the above example embodiments, but various modifications are possible, and it goes without saying that the modifications are also included within the scope of the example embodiments.

As described above, the voice processing device and the like according to one aspect of the example embodiments have the effect of enhancing the accuracy of attribute identification of a person, and are useful as a voice processing device and the like and an attribute identifying device.

### [Reference signs List]

- 100: Voice processing device
- 110: Voice section detecting unit
- 120: Speaker feature vector calculating unit
- 130: First attribute identifying unit
- 140: Second attribute identifying unit

## Claims

1. An attribute identifying device comprising:
a first attribute identifying means identifying, based on a biological signal, first attribute information, which is a range of specific attribute values, from the biological signal; and
a second attribute identifying means identifying second attribute information, which is specific attribute information, from the biological signal and the first attribute information.

2. The attribute identifying device according to claim 1, wherein
the second attribute identifying means identifies, as the second attribute information,
at least one of a specific attribute value or a range of attribute values more limited than the range identified by the first attribute identifying means.

3. The attribute identifying device according to claim 1 or 2, wherein
the first attribute identifying means divides attribute values in two or more patterns in such a way that boundary values in ranges of the attribute values are different from each other, and identifies, as the first attribute information, a range of the attribute values in each division.

4. The attribute identifying device according to any one of claims 1 to 3, wherein
the second attribute identifying means
identifies, as tentative attribute information, a range of attribute values from the biological signal and the first attribute information, and
identifies the second attribute information from the biological signal and the tentative attribute information.

5. The attribute identifying device according to any one of claims 1 to 4, further comprising
a speaker feature vector calculating means calculating speaker feature vectors representing individuality of a speaker based on a voice signal representing a voice, which is the biological signal, wherein
the first attribute identifying means identifies the first attribute information from the speaker feature vectors, and
the second attribute identifying means identifies the second attribute information from the speaker feature vectors and the first attribute information.

6. The attribute identifying device according to any one of claims 1 to 5, wherein
the specific attribute information is
information representing at least one of age, sex, physique, emotion, and character of a person identified from the biological signal.

7. An attribute identifying method comprising:
identifying, based on a biological signal, first attribute information, which is a range of specific attribute values, from the biological signal; and
identifying second attribute information, which is specific attribute information, from the biological signal and the first attribute information.

8. A program storage medium storing a program that causes a computer to execute:
a process of identifying, based on a biological signal, first attribute information, which is a range of specific attribute values, from the biological signal; and
a process of identifying second attribute information, which is specific attribute information, from the biological signal and the first attribute information.
